# EUROPEAN PATENT SPECIFICATION

(11) **EP 2 390 246 B1**
(45) Date of publication and mention of the grant of the patent: **29.08.2018**
(21) Application number: 11166961.0
(22) Date of filing: 20.05.2011
(51) Int. Cl.: C07C 227/04

(54) **Process for the preparation of aminaphtone**
Verfahren zur Herstellung von Aminaphton
Procédé pour la préparation d'aminaphtone

(30) Priority: 27.05.2010 IT MI20100960
(43) Date of publication of application: 30.11.2011
(73) Proprietor: Laboratori Baldacci S.P.A., 56124 Pisa (IT)
(72) Inventor: Baldacci, Massimo, I-56124, Pisa (IT)
(74) Representative: Pistolesi, Roberto

(56) References cited:
- US-A- 3 639 432
- CARLO ERBA REAGENTI: "Sustainable (Green) Chemistry Green Solvents", , 2008, XP002617746, Retrieved from the Internet: URL:http://www.carloerbareagenti.com/Repos itory/Download/pdf/Catalogue/EN/catchem100 _sez2_green_en.pdf [retrieved on 2011-01-21]
- Anonymous: "1,3-Dioxolane (646-06-0)", , 2008, XP002617747, Retrieved from the Internet: URL:http://www.chemicalbook.com/ChemicalPr oductProperty_EN_CB5712494.htm [retrieved on 2011-01-21]

## Description

Herein described is a new process for the preparation of aminaphtone with high purity. Said process uses solvents with low toxicity, mild reaction conditions - such as temperature and pressure - within a short time.

### State of the art

Capillary fragility is a very common problem, in particular in the female population. Uptake of anticoagulant drugs, infections, deficiency of some vitamins, heredity, can determine a lower capillary resistance.

The therapeutic use of aminaphtone of Formula 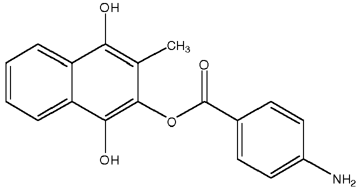 i.e. of the 2-hydroxy-3-methyl1,4-naphthohydroquinone-2-p-aminobenzoate, as a vasoprotective drug, has been known over the years; in scientific literature there are indicated studies which reveal the efficiency thereof as modulator/normaliser of the alteration of capillary vessels in particular pathologic conditions such as chronic venous insufficiency (see references 1, 2, 3).

More recent studies have revealed that aminaphtone is also efficient against disorders related to an endothelial damage of the arterial-venous microcircle with loss of vessel integrity and the formation of a potent endogenous vasoconstrictor (see references 4, 5). Up to date the preparation of aminaphtone was conducted according to what is indicated in the patent US 3639432. The synthesis in two steps first provides for the formation of an ester bond between 2-hydroxy-3-methyl-1,4-naphthohydroquinone and a p-nitrobenzoyl halide in benzene, and subsequent catalytic hydrogenation under pressure in dioxane to obtain the final product. This preparation is exemplified in the scheme 1 below.

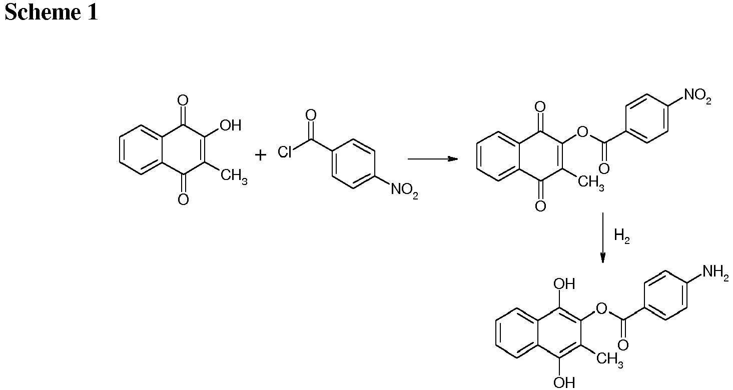

The use of toxic solvents and drastic conditions make this method of preparation scarcely suitable for an industrial scale. Furthermore, the aminaphtone obtained through this process, has an inadequate degree of purity for use as a drug. Actually, alongside aminaphtone, it rapidly forms an impurity corresponding to a product of over-oxidation of formula:

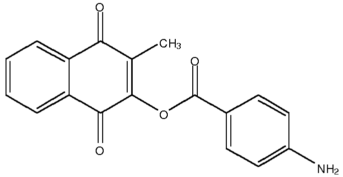

Thus, there arises the need of providing a new process for the synthesis of aminaphtone, which uses less toxic solvents, milder reaction conditions and which occurs within short periods of time to prevent the formation of impurity in oxidized form.

### Description of the figures

Figure 1: HPLC of aminaphtone obtained through the process of the present invention
Figure 2: HPLC of aminaphtone obtained through the known process (US3639432)

### Description of the invention

An object of the present invention is a new process for the preparation of aminaphtone , having high purity, suitable for synthesis on industrial scale.

The "high purity" according to the present invention, is used to indicate purity higher than 95%, in weight.

The present invention regards a process for the preparation of aminaphtone, comprising the catalytic hydrogenation, under low pressure of hydrogen, of the compound of formula (II) 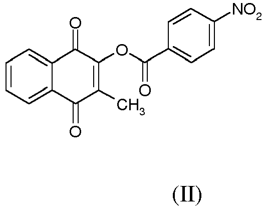 in presence of a catalyst, in dioxolane, characterised in that said catalyst is palladium on carbon with 10% to 50% of water.

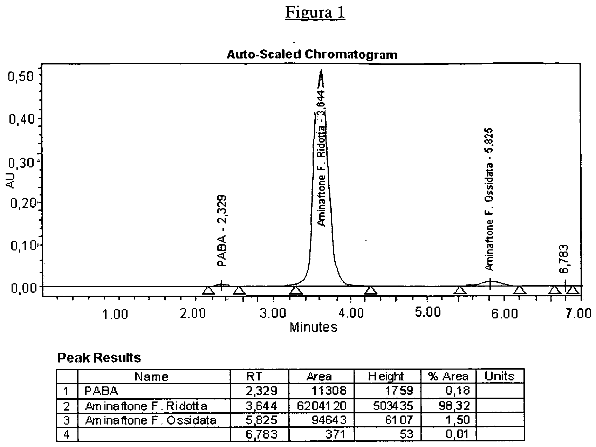

The aminaphtone, obtained according to the process of the present invention, has a purity of at least 98%, as indicated in the chromatogram of the Figure 1. This high purity makes it particularly suitable for use as an active ingredient in the preparation of a drug.

It is obtained through catalytic hydrogenation of the compound of formula (II) 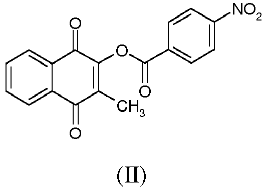 in dioxolane, i.e. in 1,3 dioxacyclopentane, so that the final product is well soluble in the working conditions.

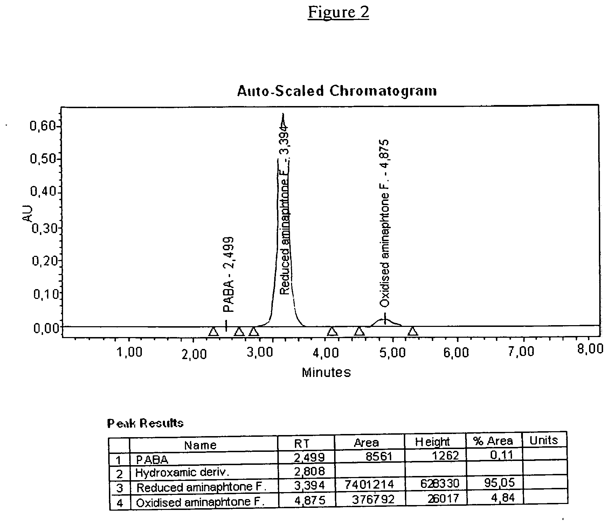

The reaction occurs in hydrogen atmosphere, at a pressure comprised between 10.1325 kPa (0.1 atm) and 101.325 kPa (1 atm, namely atmospheric pressure), preferably between 20.265 kPa (0.2 atm) and 50.6625 kPa (0.5 atm) and in presence of a catalyst, characterised in that said catalyst is palladium on carbon with 10% to 50% of water. A temperature suitable for conducting said reaction is comprised between ambient temperature and reflux temperature of the solvent, preferably the temperature is comprised between 30 and 60°C, even more preferably the reaction is conducted at about 45°C. In these conditions, the percentage of oxidised product that is formed is 1.50% in weight (see in figure 1). This percentage is 3 times lower than the one obtained with the process of the prior art (see in figure 2). Actually, with the process of the present invention, the reaction times are considerably reduced and the reaction of hydrogenation occurs in about 30 minutes.

The compound of Formula (II) is prepared according to the methods well known to the man skilled in the art, such as for example those described in US 3639432; in particular through reaction between 2-hydroxy-3-methyl-1,4-naphthohydroquinone and p-nitrobenzoyl chloride (reagents commercially available). Said reaction occurs preferably in toluene, in presence of a base, for removing the developed hydrochloric acid. A preferred base of the present invention is an organic base, preferably pyridine. The reaction sub-products are easily eliminated by washing with water.

Thus, the method of the present invention allows the preparation of aminaphtone, having high purity, using solvents with lower toxicity, mild reaction conditions, lower reaction times and better handling of the reagents.

These factors contribute to improving the safety of the operators in the production process and allow better compliance with the continuous evolution of the regulations within the pharmaceutical industry (which, for example, do not allow the use of the benzene in the synthesis of active ingredients).

The previous description, just like the illustrating example that follows, will allow observing that the method of the present invention attains the previously mentioned object and therefore has a high industrial interest.

### Example 1: Synthesis of the compound of Formula (II)

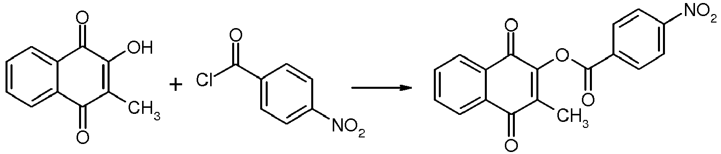

20 litres of toluene and 2.86 kg of 2-hydroxy-1,4-naphthoquinone are introduced into a 40 litre steel reactor. 3.02 kg of p-nitrobenzoyl chloride are added and the reaction mixture is heated up to 70-80°C. Pyridine (1.46 kg) is added. The temperature rises spontaneously due to the exothermic effect of the esterification reaction. The mixture is maintained between 80°C and 90°C for one hour. After cooling the product is in solid form and it is separated from the solution by filtering. The obtained solid is washed thoroughly with hot water until the washing water is free of chlorides. The obtained intermediate is dried in a fluidised bed at 50-60°C obtaining a 4 kg yield.

### Example 2: Synthesis of aminaphtone

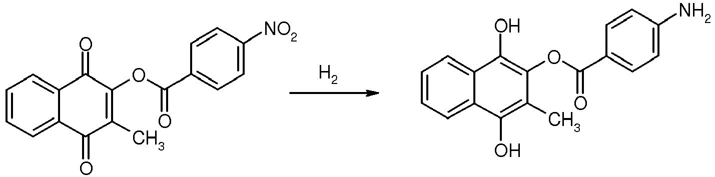

1 kg of the obtained intermediate is transferred into an apparatus for hydrogenation for the reduction reaction. 0.2 kg of palladium on carbon with 10% to 50% of water (corresponding to 0.1 kg of anhydride product, i.e. 10 g of palladium) are added. All the feeds are conducted under nitrogen to preserve the reagents and the catalyst. Nitrogen is then eliminated through a vacuum pump and hydrogen is introduced, maintaining the pressure comprised between 20.265 kPa and 50.6625 kPa (0.2 and 0.5 atm). The temperature tends to rise and it is kept at mild values (about 45°C) by cooling. The reaction continues until hydrogen consumption occurs, then it is considered completed (the pressure, increase to the value of 101.325 kPa (1 atm), it does not vary after 30 minutes, confirming that there is no further absorption of gas). The reaction mixture is filtered under nitrogen to eliminate the catalyst and the obtained solution is evaporated under vacuum at 55-60°C. The obtained product is suspended in a solution of sodium hydrosulfite (8%) and subsequently washed with water until the washing water is free of sulfates. The final product (98.32% purity) is recovered by drying at 50-70°C after filtration.

HPLC chromatograms of two batches of aminaphtone, produced through the process described in the prior art in figure 1 and through the process subject of the present invention in figure 2 are annexed by way of example. It may be observed that the percentage of oxidized product (the main impurity of this product) is considerably lower.

### Bibliography

1. Gelso E., Corradetti R.
   Etiopathogenesis of the chronic venous insufficiency: actuality of aminaphtone (Capillarema) in microcircle alterations
   *Farmaci e terapia, Vol. XXI, 1*/*2, 35-42, 2004*
2. : Villaverde C.A. et al. Modification of the vascular permeability with Aminaphtone Rev. Farmacol. Clin. Exp., 6, 9-14, 1989
3. Castelli P. et al. Treatment with Aminaphtone in the complicated chronic venous insufficiency Flebolinfologia 1, 241-244, 1988
4. Scorza R. et al. Effects of aminaftone 75 mg TID on soluble adhesion molecules: a 12-week, randomized, open-label pilot study in patients with systemic sclerosis Clin. Ther. 30, 924-929, 2008
5. Scorza R. et al. Aminaftone enanches Iloprost beneficial effects in patients with systemic sclerosis and recurrent ulcers. 2009 ACR/ARHP Ann. Scient. Meeting Philadelph. P. A. October 16th-21st

## Claims

1. Process for the preparation of aminaphtone, comprising the catalytic hydrogenation of the compound of formula (II) 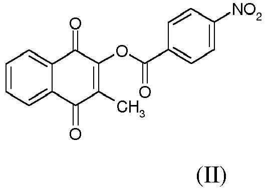 in presence of a catalyst, in dioxolane,
**characterised in that** said catalyst is palladium on carbon with 10% to 50% of water.

2. Process according to claim 1, **characterised in** being performed at a hydrogen pressure comprised between 10.1325 kPa (0.1 atm) and 101.325 kPa (1 atm), preferably between 20.265 kPa (0.2 atm) and 50.6625 kPa (0.5 atm).

3. Process according to claim 1, **characterised in that** the hydrogenation temperature is comprised between ambient temperature and reflux temperature of the dioxolane.

4. Process according to claim 3, **characterised in that** said temperature is comprised between 30 and 60°C, preferably of about 45°C.

5. Process according to claim 1, **characterised in that** hydrogenation occurs in about 30 minutes.

6. Process according to any one of the preceding claims, **characterised in that** said compound of formula (II) is prepared through reaction between 2-hydroxy-3-methyl-1,4-naphthohydroquinone and p-nitrobenzoyl chloride, in presence of a base, in toluene.

7. Process according to claim 6, **characterised in that** said base is an organic base, preferably pyridine.

## Patentansprüche

1. Verfahren zur Herstellung von Aminaphton, umfassend die katalytische Hydrierung der Verbindung der Formel (II) 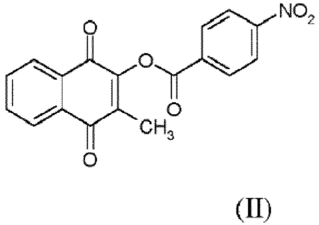 in Gegenwart eines Katalysators in Dioxolan, **dadurch gekennzeichnet, dass** der Katalysator Palladium auf Kohlenstoff mit 10% bis 50% Wasser ist.

2. Verfahren nach Anspruch 1, **dadurch gekennzeichnet, dass** es bei einem Wasserstoffdruck zwischen 10,1325 kPa (0,1 atm) und 101,325 kPa (1 atm), vorzugsweise zwischen 20,265 kPa (0,2 atm) und 50,6625 kPa (0,5 atm) durchgeführt wird.

3. Verfahren nach Anspruch 1, **dadurch gekennzeichnet, dass** die Hydrierungstemperatur zwischen Umgebungstemperatur und Refluxtemperatur des Dioxolans liegt.

4. Verfahren nach Anspruch 3, **dadurch gekennzeichnet, dass** die Temperatur zwischen 30 und 60'C, vorzugsweise bei etwa 45°C, liegt.

5. Verfahren nach Anspruch 1, **dadurch gekennzeichnet, dass** die Hydrierung in etwa 30 Minuten erfolgt.

6. Verfahren nach einem der vorstehenden Ansprüche, **dadurch gekennzeichnet, dass** die Verbindung der Formel (II) durch Umsetzung zwischen 2-Hydroxy-3-methyl-1,4-naphthohydrochinon und p-Nitrobenzoylchlorid in Gegenwart einer Base in Toluol hergestellt wird.

7. Verfahren nach Anspruch 6, **dadurch gekennzeichnet, dass** die Base eine organische Base, vorzugsweise Pyridin, ist.

## Revendications

1. Procédé pour la préparation d'aminaphtone, comprenant l'hydrogénation catalytique du composé de formule (II) 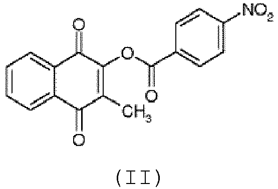 en présence d'un catalyseur, dans du dioxolane,
**caractérisé en ce que** ledit catalyseur est du charbon palladié avec 10 % à 50 % d'eau.

2. Procédé selon la revendication 1, **caractérisé en ce qu'**il est mis en oeuvre sous une pression d'hydrogène comprise entre 10,1325 kPa (0,1 atm) et 101,325 kPa (1 atm), de préférence entre 20,265 kPa (0,2 atm) et 50,6625 kPa (0,5 atm) .

3. Procédé selon la revendication 1, **caractérisé en ce que** la température d'hydrogénation est comprise entre la température ambiante et la température de reflux du dioxolane.

4. Procédé selon la revendication 3, **caractérisé en ce que** ladite température est comprise entre environ 30 et 60°C, de préférence est d'environ 45°C.

5. Procédé selon la revendication 1, **caractérisé en ce que** l'hydrogénation a lieu en environ 30 minutes.

6. Procédé selon l'une quelconque des revendications précédentes, **caractérisé en ce que** ledit composé de formule (II) est préparé par réaction entre de la 2-hydroxy-3-méthyl-1,4-naphtohydroquinone et du chlorure de p-nitrobenzoyle, en présence d'une base, dans du toluène.

7. Procédé selon la revendication 6, **caractérisé en ce que** ladite base est une base organique, de préférence la pyridine.
